(19) Europäisches Patentamt

European Patent Office

Office européen des brevets

(11) Veröffentlichungsnummer: **0 350 810 B1**

(12) **EUROPÄISCHE PATENTSCHRIFT**

(45) Veröffentlichungstag der Patentschrift: **29.09.93**

(21) Anmeldenummer: **89112446.3**

(22) Anmeldetag: **07.07.89**

(51) Int. Cl.⁵: **C07K 1/14**, C12P 21/02, //C07K7/10,(C12P21/02, C12R1:45)

(54) **Verfahren zur Gewinnung, Isolierung und Reinigung von Epidermin.**

(30) Priorität: **15.07.88 US 219698**

(43) Veröffentlichungstag der Anmeldung:
**17.01.90 Patentblatt 90/03**

(45) Bekanntmachung des Hinweises auf die Patenterteilung:
**29.09.93 Patentblatt 93/39**

(84) Benannte Vertragsstaaten:
**AT BE CH DE ES FR GB GR IT LI LU NL SE**

(56) Entgegenhaltungen:
**EP-A- 0 027 710**
**EP-A- 0 181 578**

**CHROMATOGRAPHIE, vol. 24, 1987; H.-P. FIEDLER et al., pp. 433-438&NUM;**

(73) Patentinhaber: **Dr. Karl Thomae GmbH**

**D-88397 Biberach(DE)**

(72) Erfinder: **Werner, Rolf-Günter, Dipl.Mikrobiol.Dr.**
**Hugo Häring-Strasse 72**
**D-7950 Biberach 1(DE)**
Erfinder: **Zähner, Hans, Prof. Dr.**
**Im Hopfengarten 13**
**D-7400 Tübingen(DE)**
Erfinder: **Jung, Günther, Prof.Dipl.Chem.Dr.**
**Ob der Grafenhalde 6**
**D-7400 Tübingen(DE)**
Erfinder: **Hörner, Thomas, Dipl.Biol.**
**Ouenstedtstrasse 34**
**D-7400 Tübingen(DE)**
Erfinder: **Kellner, Roland, Dipl.Chem.**
**Dr. H. Winter-Strasse 17**
**D-6148 Heppenheim/B(DE)**
Erfinder: **Fiedler, Hans-Peter**
**Bühler Rathausplatz 12**
**D-7400 Tübingen 4(DE)**

## Beschreibung

Die Erfindung bezieht sich auf Verfahren zur Gewinnung des Polypeptides Epidermin.

Das Antibiotikum Epidermin ist bekannt durch die Europäische Patentanmeldung 85 113 908.9 (Veröffentlichungsnummer 0 181 578).

Dort wird ein Verfahren zur Gewinnung, Isolierung und Reinigung dieser Substanz beschrieben, ausgehend von einer Kulturbrühe, die unter Mitwirkung eines resistenten Stammes von Staphylococcus epidermidis erhalten wurde. Der resistente Stamm wurde am 26.10.1984 unter der Nummer DSM 3095 bei der "Deutsche Sammlung von Mikroorganismen" hinterlegt. Die aktive Komponente wird zur Isolierung entweder durch Extraktion des von Zellen und Kalk befreiten Kulturfiltrats mit n-Butanol angereichert, der Butanolextrakt wird evaporiert, der Rückstand in Methanol gelöst und in eine überschüssige Menge kalten Diethylethers zur Abtrennung der lipidischen Begleitstoffe eingerührt, wobei die Aktivität im Niederschlag verbleibt, oder das zentrifugierte Kulturfiltrat wird zu Anreicherung des Epidermins an Amberlite® XAD-8 oder an verwandte Typen dieses Polymers auf Acrylesterbasis (Firma Serva) adsorbiert, wobei das adsorbierte Epidermin mit Methanol/konz. Salzsäure (99:1) vom Harz gelöst und aus der salzsauren Methanollösung nach Neutralisation mit Ammoniak durch Eindampfen isoliert wird. Eine sich anschließende Chromatographie des Amberlite® XAD-Eluats bzw. des von Lipiden befreiten Butanolextrakts an Sephadex LH-20 mit Methanol/Essigsäure (95:5) trennt eine große Zahl von kleinen Peptiden, Aminosäuren und Salzen aus dem Medium vom Antibiotikum ab. Bei einer sich anschließenden multiplikativen Gegenstromverteilung nach Craig bleibt in einer ersten Flüssig-Flüssig-Verteilung mit dem System n-Butanol/Essigester/0,1 N Essigsäure (3:1:3) das Antibiotikum am Start sitzen. Bei einer zweiten Craig-Verteilung mit dem neutralen System 2-Butanol/0,05 N Ammoniumacetat (1:1) findet sich das Antibiotikum in der Mitte der Apparatur. Das Ammoniumacetat wird durch Lyophilisation am Hochvakuum entfernt, das gebildete Epidermin fällt nach Gefriertrocknung als ein in allen verwendeten Dünnschichtsystemen einheitliches, weißes Pulver an. Auf diese Weise lassen sich aus 80 Liter Kulturfiltrat (bei Adsorption an Amberlite® XAD-8, Gelchromatographie an Sephadex LH-20 und multiplikative Verteilung nach Craig) 2,6 g lyophylisiertes Epidermin gewinnen.

Die Zucht des Produzenten Staphylococcus epidermidis DSM 3095 erfolgt, wie dort beschrieben, aerobisch bei 37°C in einem Komplexmedium der Zusammensetzung 2 bis 4 % Fleischextrakt, 1 bis 3 % Malzextrakt und 0,25 bis 1 % $CaCO_3$ oder 0,25 bis 0,5 % $Ca(OH)_2$ (Gewichtsprozente).Das Maximum an antibiotischer Aktivität wird dabei nach 18 bis 23 Stunden erreicht.

Es wurde gefunden, daß sich das Antibiotikum Epidermin in wesentlich höheren Ausbeuten und auf einfachere Weise dadurch gewinnen läßt, daß das gebildete Antibiotikum in dem Kulturfiltrat oder in der Kulturbrühe, die noch den Mikroorganismus enthält, an Amberlite® XAD-1180 oder Amberlite® XAD-16 oder verwandte Polymertypen auf Basis von Styrol-divinyl-copolymerisatenentweder über eine Kolonne oder, im Falle der Kulturbrühe, auch durch schubweises Eintragen des Harzes adsorbiert wird. Die Wirkkomponente wird durch Elution mit Methanol/verdünnter insbesondere 0,01 N Salzsäure (9:1,v:v) vom Harz losgelöst, das Eluat auf einen pH-Wert von 5,3 bis 5,8 eingestellt und auf einen schwachen Kationenaustauscher, wie Amberlite® IRC-50 oder verwandte Harze, wie z. B. Amberlite® IRC-84 gegeben, wobei der Ionenaustausch in einer Säule oder auch durch schubweises Eintragen erfolgen kann, im letzteren Fall vorzugsweise durch zweimaliges Zugeben einer Menge von jeweils 3 Volumenprozent des Harzes zum XAD-Eluat innerhalb von einer Stunde. Nach der Adsorption wird das Harz in eine Säule eingegeben, nichtgebundene Substanzen werden anschließend mit einer 0,05 N Natriumphosphatpufferlösung in Wasser bei pH 7,0 ausgewaschen, das Epidermin wird dann mit einer Lösung von 80 % des vorstehend genannten Natriumphosphatpuffers, die 1,5 N bezüglich Kochsalz ist und 20 Vol.-% Methanol enthält, bei einem pH-Wert zwischen 6,0 und 8,0 vorzugsweise 7,0 eluiert.

Zur Entsalzung stellt man das Eluat auf einen pH-Wert von 6,0 und gibt dieses schubweise zur Adsorption auf ein Harz des obengenannten Styrol-divinyl-copolymerisat-Typs, z. B. auf Amberlite® XAD-1180. Die Salze werden mit Wasser ausgewaschen und das gebundene Epidermin anschließend mit Methanol/50 %-ige Essigsäure (9:1, v:v) eluiert. Nach Entfernung des Lösungsmittels und Gefriertrocknung erhält man ein lyophilisiertes Roh-Epidermin. Aus diesem läßt sich durch präparative HPLC-Chromatographie (high performance liquid chromatography) mit Hilfe von Nucleosil 100 C-18 (10 µm) oder Lichro-Sorb RP Select B reines Epidermin isolieren. Das Epidermin wird zu diesem Zweck schrittweise ausgewaschen, zuerst mit einem Lösungsmittel A, bestehend aus Wasser mit 1 Gew.-% Ameisensäure, dann mit einem Lösungsmittel B, bestehend aus Methanol/Wasser (80:20, v:v) mit 1 Gew.-% Ameisensäure.

Verfahrenstechnisch läßt sich die Epidermin-Erzeugung auf dem Wege einer "Batch"-Fermentation, einer hierzu verwandten "Feeding"-Fermentation, bei welcher einzelne Stoffe während der Fermentation zugegeben werden, einer Fermentation mit diskontinuierlicher Adsorption des gebildeten Epidermins als "on-line"-Adsorption und einer on-line-Adsorption, die kontinuierlich verläuft, durchführen.

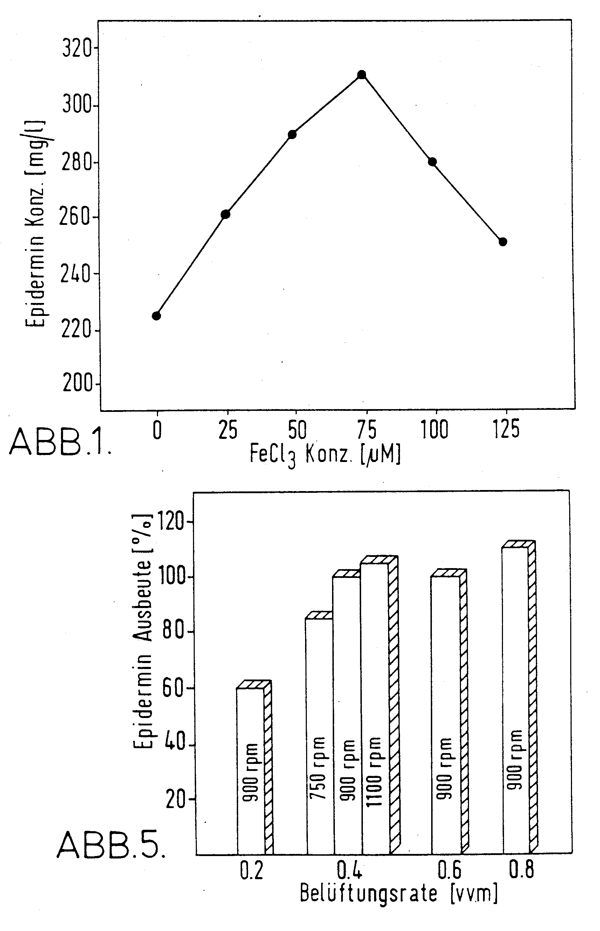

Bei der ansatzweisen sogenannten "Batch"-Fermentation wurden die besten Ergebnisse mit einer Nährstofflösung der folgenden Zusammensetzung erreicht: 3,3 % Fleischextrakt, 3 % Malzextrakt, 0,37 % Calciumhydroxid (Gew.-%). Vorteilhaft wirkt sich auf die Ausbeute ein Zusatz von 1 bis 6 Gew.-% Alkalichloride, wie Natriumchlorid, Kaliumchlorid und von 0,001 bis 0,002 Gew.-% Eisenionen, z. B. in Form von $FeCl_3$ und/oder $FeSO_4$, desweiteren von Ammoniumchlorid oder -sulfat (zwischen 57 und 200 m molar) aus; vorzugsweise angewandte Konzentrationen sind 3 Gew.-% Natriumchlorid und 0,00125 Gew.-% Ferrichlorid. Der Einfluß anfänglicher Konzentrationen von 3 % Kochsalz und unterschiedlicher Konzentrationen von Ferrichlorid auf die Epiderminproduktion wird durch Abb. 1 gezeigt. Von allen C-Quellen gab Maltose nach Malzextrakt die besten Ergebnisse. Es empfiehlt sich Glucose nur in Verbindung mit anderen C-Quellen zuzusetzen. Eine Kombination von Laktose mit Maltose oder Galaktose mit Maltose gab ebenfalls gute Ergebnisse. Eine Kombination von Glukose mit Maltose oder Laktose oder Galaktose erbrachte die gleiche Ausbeute wie der Malzextrakt. Alle anderen, konventionellen C-Quellen erbrachten keine oder nur eine geringe Epiderminproduktion.

Die Fermentation erfolgt unter guter Belüftung bei Temperaturen zwischen 34 und 37°C vorzugsweise 36°C. Den besten Produktionsverlauf erhält man, wenn der pH-Wert vor der Fermentation bei 6,0 bis 7,0 liegt. Bei Fehlen von Carbonaten oder Hydroxiden zweiwertiger Kationen, wie Calciumcarbonat oder Calciumhydroxid, findet nur geringe Produktion statt. Nach der Zugabe von beispielsweise Calciumcarbonat zeigt der pH-Wert einen charakteristischen Verlauf mit Abfall in den sauren Bereich, dabei findet nur geringe Produktion statt.

Beim anschließendem Anstieg des pH-Wertes in den alkalischen Bereich setzte die Produktion ein. Anstelle von Calciumcarbonat kann auch Magnesiumcarbonat verwendet werden, Calciumhydroxid lieferte bessere Ergebnisse als Calciumcarbonat. Mit 50 mM Calciumhydroxid konnte die Produktion gegenüber 25 mM Calciumcarbonat noch etwas gesteigert werden. Bei der Verwertung von C-Quellen (Zucker) durch den Stamm kommt es zur Bildung organischer Säuren wie z. B. Essigsäure, die durch zweiwertige Kationen komplexiert werden, wobei gleichzeitig eine Abpufferung des Mediums erfolgt. Die Aktivitätszunahmen zum Zeitpunkt der maximalen Produktion durch den Stamm DSM 3095 ergaben sich, ermittelt durch den Plattendiffusionstest (Bestimmung des Hemmhofdurchmessers in mm gegen Micrococcus luteus ATCC 9341), unter Benutzung einer kalibrierten Linie, wobei die Aktivität in einer Brain-Heart-Infusionsnährlösung gemäß der EP-A-0 027 710 gleich 100 % gesetzt wurde, wie folgt:

nach Verfahren gemäß dem Stand der Technik:

a.) Brain-Heart-Infusion-Agar
gemäß Ep-A-0 027 710                                      100 %

b.) 3 % Fleischextrakt, 2 % Malzextrakt,
25 mM Caliziumcarbonat
gemäß EP-A-0 181 578                                      200 %

c.) 3 % Fleischextrakt, 2 % Malzextrakt,
50 mM Calciumhydroxid
gemäß EP-A-0 181 578                                      320 %

nach den erfindungsgemäßen Verfahren:

d.) 3,3 % Fleischextrakt, 3 % Malzextrakt,
50 mM Calciumhydroxid                                    440 %

e.) 3,3 % Fleischextrakt, 3 % Malzextrakt,
50 mM Calciumhydroxid, 3 % Natriumchlorid,
75 µM Eisen(III)chlorid                      1 720 %

f.) 3,3 % Fleischextrakt, 3 % Malzextrakt,
50 mM Calciumhydroxid, zusätzliche Zugabe
von Glucose, $KH_2 PO_4$ und Ammoniumchlorid                                      1 950 %

g.) 3,3 % Fleischextrakt, 3 % Malzextrakt,
50 mM Calciumhydroxid, unter zusätzlicher
"on-line"-Adsorption des erzeugten Epidermins
nach dem ersten Isolationsschritt, weitere
Zusatzstoffe siehe unter Punkt f            2 780 %.

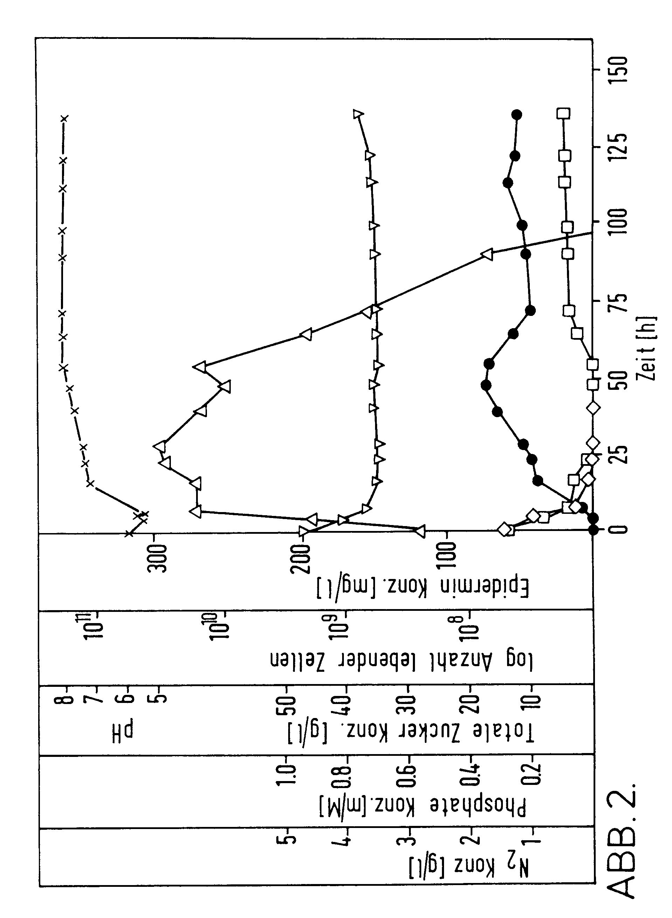

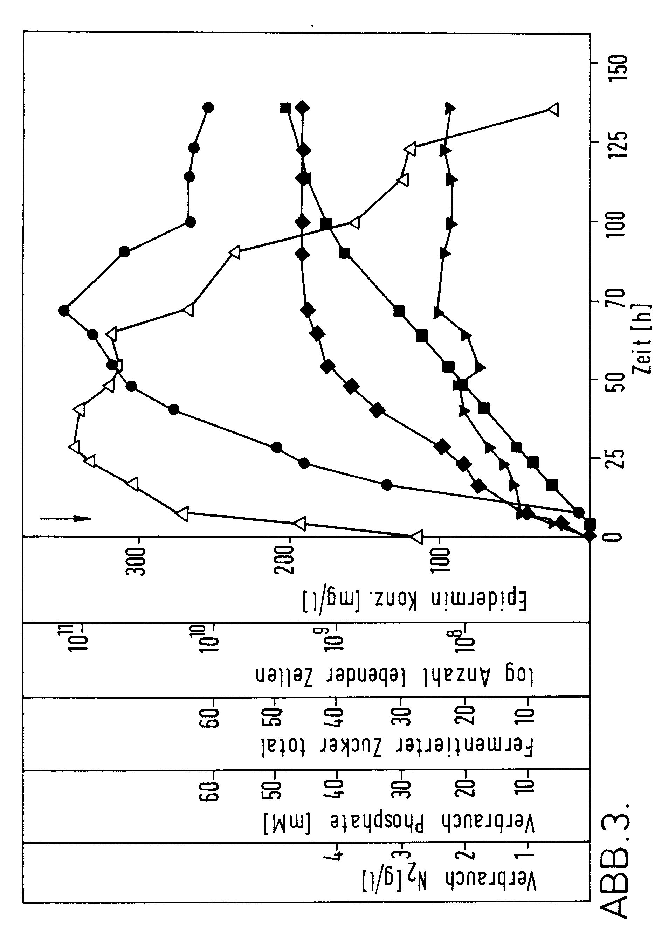

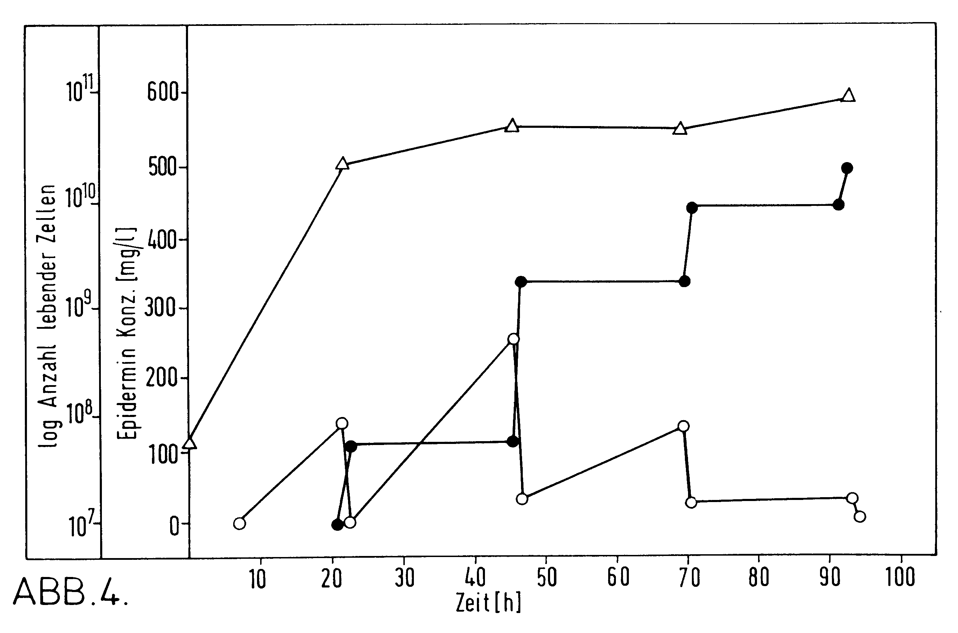

Die Abb. 2 zeigt den Verlauf der Fermentation wie vorstehend unter d) beschrieben; die Abb. 1 zeigt die Abhängigkeit der Epiderminproduktion von der Zugabe von 3 % Natriumchlorid und von verschiedenen Mengen an Eisen(III)chlorid wie unter e) angegeben. Alle vorstehend genannten Prozentangaben betreffen Gewichtsprozente. Die Abb. 3 zeigt den Verlauf der Fermentation wie unter Punkt f) beschrieben, die Abb. 4

EP 0 350 810 B1

denselben Verlauf unter Einschaltung der on-line Adsorption, wie oben unter Punkt g) angegeben.

Ein Vergleich der mit Hilfe des Platten-Diffusions-Tests oder der HPLC gewonnenen Werte zeigt bei der Anwendung des erfindungsgemäßen Verfahrens mit seinen Varianten im Vergleich zu den an sich bekannten Verfahren ein signifikantes Anwachsen der Epidermin-Ausbeuten, nämlich von 320 % auf bis zu 2780 %.

Im folgenden sollen die einzelnen Schritte und Bedingungen für die Durchführung des erfindungsgemäßen Verfahrens genauer beschrieben werden.

Der produzierende Stamm läßt sich am besten durch Tiefgefrieren (-18°C) in einem Medium, das 3,3 Gew.-% Fleischextrakt, 3 Gew.-% Malzextrakt, 0,37 Gew.-% Calciumhydroxid in 40 Gew.-%, Glyzerin (der Rest ist Wasser) enthält, lagern. Für jede Fermentation läßt man eine Praekultur 18 Stunden lang bei 36°C auf einem Agar-Medium (pH 7,2 bis 7,4), das pro Liter 8 g Lab Lemco Pulver (Firma Oxoid), 10 g Pepton, 3 g Kochsalz, 2 g $Na_2HPO_4$, 15 g Agar und 10 g sterilisierte Glucose enthält, wachsen.

Die Fermentation läßt sich in dazu geeigneten Schüttelkolben durchführen, zur Herstellung größerer Substanzmengen können auch Fermenter von 200 Liter und mehr verwendet werden.

Für Kolbenversuche werden 500 ml Erlenmeyerkolben mit einem seitlichen Einstich verwendet. Die Kolben werden mit 100 ml Nährlösung gefüllt und 20 Minuten bei 121°C autoklaviert. Als Impfmaterial dient 1 % einer 8 Stunden alten Vorkultur. Die Inkubation erfolgte bei 36°C auf der rotierenden Schüttelmaschine bei 160 Umdrehungen pro Minute (rpm).

Für die Fermentation im 15 l Maßstab wurde ein 20 l Fermenter (Typ b 20. Braun/Melsungen bzw. Giovanola Freres, Monthey, Schweiz; mit Umwurfsystem) mit 15 l Nährlösung unter Zusatz von 0,5 ml Polyol (Propylenglykol) bestückt und in situ bei 121°C 30 Minuten sterilisiert. Als Impfmaterial dienten 150 ml einer 8 Stunden alten Vorkultur. Die Fermentation erfolgte bei 35 bis 37°C, 0,2 bis 0,6 vvm und 700 bis 1000 rpm, vorzugsweise bei 36°C, 0,4 vvm und 900 rpm.

Epiderminerzeugung durch "Batch"-Fermentation.

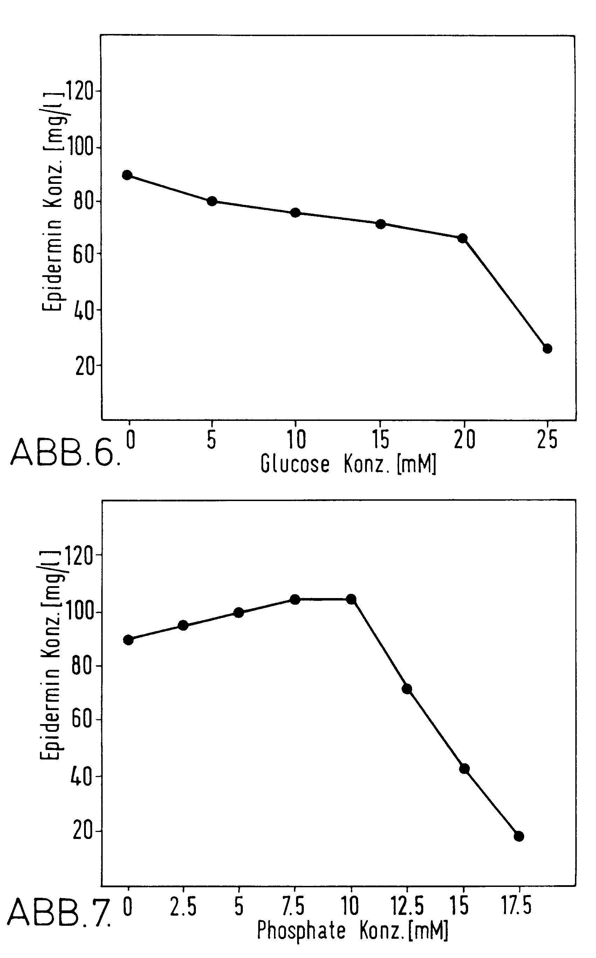

Bei den verwendeten Bioreaktoren ergab sich das beste Wachstum und die beste Ausbeute an Epidermin bei 36°C, einer Belüftungsrate von 0,4 vvm und einem Rühren mit 900 Umdrehungen pro Minute. Eine geringere Belüftung und ein langsameres Rühren verminderte die Ausbeute an Epidermin (Abb. 5). Stärkere Belüftung und Rühren führte zu einer leicht verbesserten Ausbeute aber auch zu einem extremen Schäumen. Dies rührt von der starken Oberflächenaktivität des Antibiotikums her; mechanische oder chemische Schaumunterdrückung war nicht ohne einen Verlust an Aktivität möglich.

Die Abb. 2 zeigt den Verlauf einer "Batch"-Fermentation im 20 l Maßstab mit dem stark produzierenden Stamm DSM 3095, in einer Nährlösung, enthaltend 33 g Fleischextrakt, 30 g Malzextrakt, 3,8 g Calciumhydroxid in 1 Liter. Dieser Stamm erzeugt hierin Epidermin in einer Menge bis zu 80 mg/l. Intensives Wachstum verbunden mit der Verwertung der angebotenen C-Quellen unter Acetatbildung kann an Hand des Abfalls des pH-Wertes erkannt werden. Die maximale Zellzahl wird nach 30 Stunden erreicht. Nach dieser Zeit sind die hauptsächlichen, fermentierbaren C-Quellen, wie Glukose und Maltose, erschöpft. Das zugesetzte Phosphat ist nach 8 Stunden verbraucht. Die maximale Konzentration des Antibiotikums wird nach 48 Stunden erreicht und beträgt 80 mg/l.

Sowohl die Zugabe von Chloriden, wie Natriumchlorid oder Kaliumchlorid, in einer Menge bis zu 70 g pro Liter und von $FeCl_3$ oder $FeSO_4$ bis zu 150 $\mu M$ erhöhten die Epiderminausbeute ohne Verlängerung des Fermentationsprozesses (Abb. 1). Eine maximale Ausbeute an Epidermin von 310 mg/l ergab sich bei gleichzeitiger Zugabe von 3 % Natriumchlorid und 75 $\mu M$ Eisen(III)chlorid zu dem Produktionsmedium.

Diese rasche Epiderminproduktion bietet eine gute Möglichkeit für die Entwicklung eines kontinuierlichen Fermentationsverfahrens mit hohen Durchsatzmengen des angewandten Mediums.

Epiderminerzeugung durch "Feeding"-Fermentationen:

Um die Ausbeute an Epidermin durch die Ausdehnung der Wachstumsphase und durch das Erreichen einer höheren Zell-Dichte erhöhen zu können, waren höhere Konzentrationen sowohl der C-Quellen als auch des zugesetzten Phosphats erforderlich. Die Erzeugung des Epidermins unterliegt einer starken C-katabolischen Repression (Abb. 6) und wird auch durch den Phosphatgehalt des Mediums mitreguliert (Abb. 7).

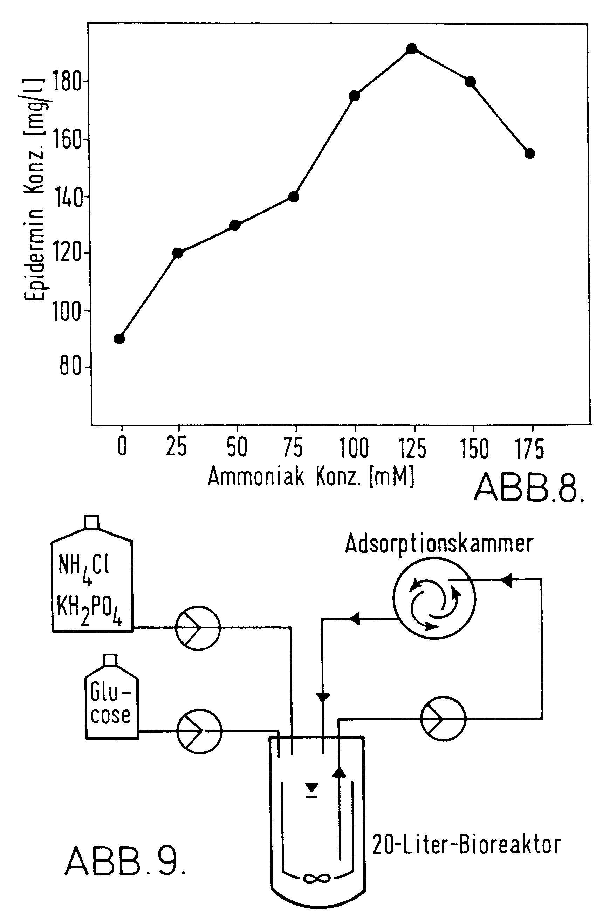

Die Stickstoffkonzentration liegt während des ganzen Verlaufs der Fermentation über 150 mM, der größte Teil dieses Gehalts kann von den Mikroorganismen nicht genutzt werden. In Flaschen-Kulturen führte die Zugabe von Ammoniumsalzen bis zu 150 mM, wie aus Abb. 8 hervorgeht (für Ammoniumchlorid), zu einer signifikanten Stimulierung der Epiderminproduktion.

5

Glucose wird primär zu Acetat metabolisiert, wie die Ansäuerung des Mediums zeigt. Werden die Zucker knapp, so werden die organischen Säuren als weitere C-Quellen benutzt, wobei der pH-Wert des Mediums in den alkalischen pH-Bereich wechselt. Die Glucose wird deshalb pH-abhängig während des Fermentationsverlaufs zugegeben. Der pH-Wert wird bei pH 6,0 gehalten, die Ansäuerung wird nicht unterdrückt.

Phosphate werden kontinuierlich zugegeben. Die Zugaberate wurde an Hand der Verbrauchsrate der Phosphate in "Batch"-Fermentationen, bei denen 10 mM Phosphat zugesetzt wurden, errechnet.

Weder die Zugabe von Glucose und Phosphat allein noch zusammen führte zu einer signifikant höheren Ausbeute an Epidermin bei der Fermentation. Der höhere Gehalt an Epidermin, der bei der pH-regulierten Glucosezugabe während der Fermentation gefunden wurde, hängt nicht von zusätzlichen C-Quellen ab, er ist bedingt durch den langsameren Abbau des Antibiotikums im sauren Medium. Dies läßt sich durch Fermentationen, bei welchen der pH-Wert durch Zugabe von Schwefelsäure konstant gehalten wurde, zeigen. Erst die Entwicklung einer kombinierten "feeding"-Fermentation mit pH-abhängiger Glucosezugabe und kontinuierlicher Zugabe von Phosphaten und Ammoniumstickstoff führte sowohl zu einer signifikanten Erhöhung der Biomasse als auch zu einer Erhöhung der Antibiotikumausbeute. Die Abb. 3 zeigt den Verlauf einer kombinierten "feeding"-Fermentation.

Die Zellmasse wächst um den dreifachen Betrag auf $2x10^{11}$ Zellen pro ml an; die maximale Epiderminausbeute beträgt 350 mg/l. Obwohl die maximale Zellkonzentration bereits nach 24 Stunden erreicht ist, wird die maximale Epiderminausbeute erst während der stationären Phase nach 72 Stunden erreicht; im Verlauf der "feeding"-Fermentation verschwindet die Identität der Wachstums- und Produktionsphase, die kennzeichnend ist für die "Batch"-Fermentation. Trotzdem bleibt die Epiderminproduktion eng verbunden mit dem Wachstum der Organismen. Erstens werden 80 % der Totalausbeute während der log-Phase produziert, zweitens ergibt sich die Biomasse durch die Anzahl der lebenden Zellen, so daß die stationäre Phase als ein ausbalancierter Zustand, gegeben durch die wachsenden und lysierenden Zellen, zu betrachten ist.

Die Zugabe von Natriumchlorid während der "feeding"-Fermentation erzeugt nicht dieselbe stimulierende Wirkung wie im Falle der "Batch"-Fermentation. Während der ersten 24 Stunden war die Produktionsrate um 40 %, die Wachstumsrate um 70 % höher als im Falle der "feeding"-Fermentation. Die spezifische Produktivität des Mikroorganismus liegt deshalb um 20 % niedriger; nach 24 Stunden hört die Epiderminproduktion auf, was entweder auf eine Nährstoff-Limitierung durch einen bis jetzt unbekannten Faktor oder auf eine Eigenvergiftung des Organismus durch auftretende metabolische Nebenprodukte zurückzuführen ist.

Die kombinierte "feeding"-Fermentation wurde auch in den 200 l-Maßstab einer Pilotanlage übertragen unter Beibehaltung derselben Belüftungsrate, Rührerbewegungsrate und derselben Zugabebedingungen. Ohne zusätzliche Optimierungen erlangte man Epiderminausbeuten in der Größenordnung von 80 bis 90 % der im 20 l-Maßstab erhaltenen Ausbeuten

Epidermingewinnung mit "on-line"-Adsorption des erzeugten Antibiotikums.

Diskontinuierliche Adsorption:

Um feed-back Hemmung und andere mögliche Einflüsse auf die produzierenden Organismen zu umgehen und um das bereits gebildete Epidermin vor dem zersetzenden Einfluß von Proteasen und Wärme zu schützen, wurde das Epidermin diskontinuierlich aus der Fermentationsbrühe während des Verlaufs der Fermentation entfernt.

Zu diesem Zwecke wurde die gesamte Fermentationsbrühe einschließlich der produzierenden Organismen unter Druck in eine kugelförmige Adsorptionskammer gesprüht, die mit Amberlite® XAD-1180 befüllt war. Dies führte zu einer sehr wirkungsvollen Verwirbelung des Harzes und zu einer raschen Adsorption des Epidermins. Die frei fließenden Harzkügelchen wurden mit einem Sieb (Durchmesser 0,25 mm) abfiltriert, wobei die Fermentationsbrühe mit der Biomasse wieder dem Reaktor zugeführt wurde (Abb. 9). Der Verlauf einer "feeding"-Fermentation mit diskontinuierlicher Adsorption des Epidermins wird durch Abb. 4 gezeigt. Die Fermentationsparameter und Zugabebedingungen waren mit den oben beschriebenen identisch. Die maximale Zellmasse wurde nach 46 Stunden mit $4.10^{10}$ Zellen erreicht, die maximale Epiderminausbeute nach dem Eluieren von Harz betrug 500 mg/l. Die Gesamtzeit der Fermentation war dieselbe wie bei der "feeding"-Fermentation.

Die erste Reinigung bzw. Rohreinigung wird durch die Einbeziehung des ersten Isolationsschrittes in das Fermentationsverfahren vereinfacht; die Ausbeute an Epidermin nach Vornahme dieses ersten Reinigungsschrittes liegt um 50 % höher im Vergleich zu der Ausbeute bei der "feeding"-Fermentation vor der Reinigung.

Kontinuierliche Adsorption:

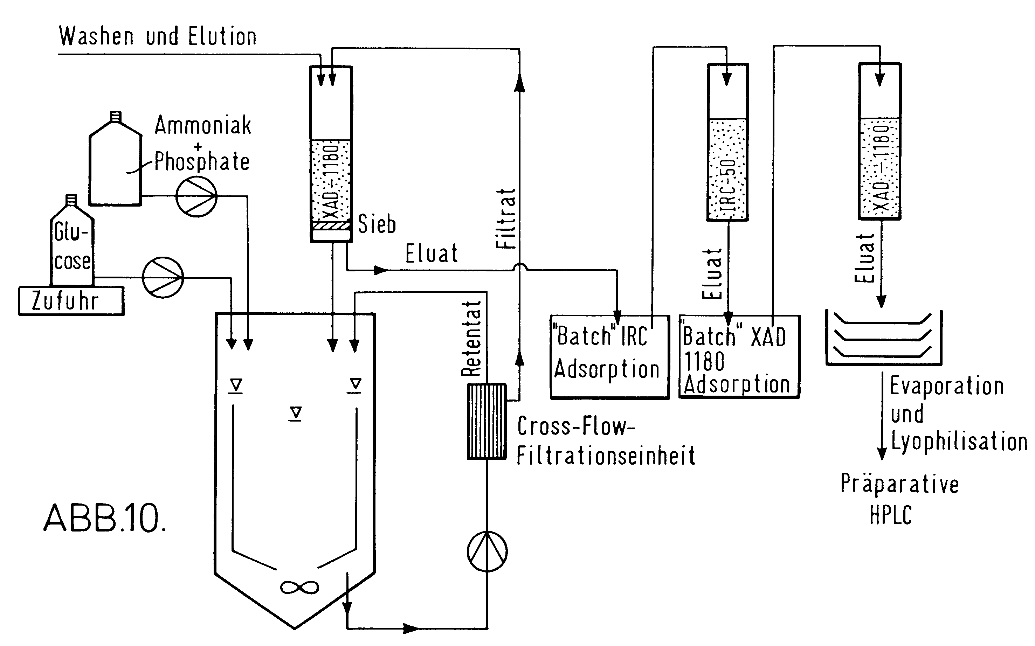

Die kontinuierliche "on-line"-Adsorption des Epidermins während des Fermentationsprozesses wird mit einer cross-flow-Filtrationsanlage erreicht. Das Retentat wird in den Reaktor zurückgeführt während das Filtrat an einer Amberlite® XAD-1180 Säule adsorbiert wird. Auch das Eluat wird in den Reaktor zurückgeführt (vgl. Abb. 10). Die Adsorption wird nach 12 bis 15 Stunden Fermentationszeit in Gang gesetzt. Maximale Epiderminausbeuten nach Loslösen des Antibiotikums vom Adsorberharz werden mit Mengen bis zu 500 mg/l nach 80 bis 90 Stunden erreicht.

Das optimierte Schema der Isolation, wie aus Abb. 10 ersichtlich, stellt eine große Errungenschaft bei dem Versuch, die Produktion von Epidermin zu steigern, dar. Letztendlich ergibt sich aus der Verbindung der erfindungsgemäßen Adsorption mit der Ionenaustauschchromatographie ein Produkt mit einer Reinheit von 80 %. Die Anwendung sowohl der Batch-, als auch der on-line-Adsorptionsverfahren führen zu einer schnellen Methode zur Reinigung des Epidermins.

Zur Kontrolle des Fermentationsverlaufes wurden zu verschiedenen Zeiten während der Fermentation steril Proben entnommen. Die Proben wurden folgendermaßen ausgewertet:

a) pH-Werte:

Messung mit einem Labor-pH-Meter (Knick pH-mV-Meter)

b) Wachstumsverlauf:

Das Wachstum konnte anhand der Zunahme der Lebendkeimzahl verfolgt werden.

Hierzu wurden 0,5 ml steril entnommene Kultur in Saline verdünnt und davon 0,1 ml auf Platten (Medium: Pepton 10 g, Fleischextrakt 8 g, Kochsalz 3 g, Dinatriumhydrogenphosphat 2 g, Glucose 10 g auf 1 Liter) ausplattiert. Nach 18 Stunden Inkubation bei 37°C konnten die Einzelkolonien ausgezählt werden.

c.) Antibiotikumkonzentration:

Die Proben wurden in einer Eppendorf-Zentrifuge 3200 2 Minuten abzentrifugiert und entweder 10 $\mu$l des Überstandes im Plattendiffusionstest oder mittels HPLC, wie nachstehend beschrieben, getestet. Parallel wurde eine Eichkurve mit bekannten Konzentrationen erstellt.

HPLC-System:

Aufspritzvolumen : 10 $\mu$l  
Fließmittel : A : Wasser mit 0,05 % 70 %-iger Perchlorsäure  
                B : Acetonitril  
Gradient :

| Minuten | A | B |
|---|---|---|
| 0 | 77,5 | 22,5 |
| 8 | 63,0 | 37,0 |
| 8,5 | 0 | 100 |
| 9,5 | 0 | 100 |
| 10 | 77,5 | 22,5 |
| 14 | 77,5 | 22,5 |

Fließrate : 2 ml/Min.  
Detektion : 210 nm  
Säule : Nucleosil 7 C-18 mit zugehöriger Vorsäule.

d) Phosphatbestimmung:

Der Phosphatgehalt im Kulturfiltrat wurde nach der Methode von Itaya und Ui (1966) in Clin. Chim. Acta 14, 361-366 gemessen.

e) Stickstoffbestimmung:

Die Stickstoffkonzentration wurde nach der Mikro-Kjeldahl-Methode mit einem automatischen Destilliergerät (Type Kjeldahlsystem II, Firma Tecator) gemessen.

f) Bestimmung der Glucose und Maltose:

Der Gehalt an Glucose und Maltose im Medium wurde enzymatisch mit einem Test-Kit der Firma Boehringer, Mannheim gemessen.

g) Acetatbestimmung:

Diese Bestimmung erfolgte gaschromatographisch nach den Angaben von Platen und Schink (1987), Arch. Microbiol. 149, 136-141.

h) Epiderminbestimmung:

Die Epiderminkonzentration in der Kulturbrühe wurde einmal durch Bioassay, das andere mal durch HPLC nach Fiedler et al., 1987, Chromatographia 24, 433-438, gemessen.

Nach Erreichen des Produktionsmaximums wurde die Kulturflüssigkeit mittels kontinuierlicher Zentrifugation (Zentrifuge: Typ LA 7lb-4, Loher & Söhne, Ruhstorf/Rott) mit 1380 rpm abzentrifugiert. Für eine optimale Abtrennung der Zellen mußte die Durchflußrate sehr niedrig gehalten werden. Eine erste Anreicherung der aktiven Komponenten wurde mittels der eingangs geschilderten Adsorption an Styrol-divinyl-copolymerisate erreicht.

Weitere Verfahren zur Adsorption des Antibiotikums, beispielsweise durch batch-Adsorption, diskontinuierliche und kontinuierliche Adsorptionsprozesse ohne und mit Abtrennung der Zellmasse wurden bereits vorne beschrieben.

Die nachfolgenden Beispiele sollen die Erfindung noch besser erläutern:

Beispiel 1

"Batch"-Fermentation mit einem Stamm des Staphylococcus epidermidis DSM 3095:

| Medium: | 3,3 Gew.-% Lab Lemco Pulver |
| | 3,0 Gew.-% Malzextrakt |
| | 0,38 Gew.-% Calciumhydroxid |
| | pH 6,5 (mit 3 N $H_2SO_4$) |
| Reaktor; | Typ b 20 (Giovanola) mit 15 l Medium |
| | Belüftung: 0,4 vvm |
| | Rührfrequenz: 900 U/min. |
| | Temperatur; 36°C |
| Ergebnis: | Max. Epiderminausbeute: 80 mg/l nach 48 Stunden; Ergebnisse siehe Abb. 2 |

Beispiel 2

"Feeding"-Fermentation mit einem Stamm des Staphylococcus epidermidis DSM 3095

| Medium: | 3,3 Gew.-% Lab Lemco Pulver |
| | 3,0 Gew.-% Malzextrakt |
| | 0,38 Gew.-% Calciumhydroxid |
| | pH 6,5 (Mit 3N $H_2SO_4$) |
| Reaktor: | Typ b 20 (Giovanola) mit 15 l Medium |
| | Belüftung: 0,4 vvm |
| | Rührfrequenz: 900 U/min. |
| | Temperatur: 36°C |

Zugabebedingungen:

Lösung 1:

Glucose 1 kg, Wasser 1 l, pH 6,0. Diese Lösung wurde pH-abhängig zugegeben, der pH-Wert der Fermentationslösung wurde auf 6,0 eingestellt.

Lösung 2:

$NH_4Cl$ 500 g, $KH_2PO_4$ 120 g, Wasser 1,5 l, pH 6,0 (eingestellt mit wasserfreiem NaOH). Die Lösung wurde konstant zugeführt mit einer Fließrate von 1 ml pro Liter und Stunde. Die Zugabe begann nach 4 Stunden.

Ergebnis:

Maximale Epiderminausbeute: 350 mg/l nach 72 Stunden. Ergebnisse siehe auch Abb. 3.

Beispiel 3

"Feeding"-Fermentation mit "on-line"-Adsorption des Antibiotikums:

| Medium: | siehe Beispiel 2 |
|---|---|
| Reaktor: | siehe Beispiel 2 |
| Adsorption: | an Amberlite® XAD-1180; 150 g Trockengewicht |
| Ergebnisse: | Maximale Epiderminausbeute: 500 mg/l nach 72 Stunden; die Epiderminausbeute wurde nach dem 1. Isolierungsschritt (siehe auch Beispiel 4) gemessen. Ergebnisse siehe auch Abb. 4. |

Beispiel 4

Isolierung und Reinigung des Epidermins aus einer 15 l Kulturbrühe nach Anwendung der "on-line"-Adsorption:

Nach "on-line"-Adsorption des Epidermins während des Fermentationsprozesses wurde das Harz (vgl. Beispiel 3) mit 150 l Wasser gewaschen und, wenn eine Adsorptionskammer benutzt wurde, in eine Säule für weiteres Waschen und Eluieren übertragen.

| Waschen: | 5 l Methanol/$H_2O$ 1:1 (v:v) |
|---|---|
| Elution: | 5 l Methanol/0,01 N Salzsäure 9:1 (v:v). |

Der pH-Wert des Eluats wurde auf 5,5 eingestellt; das Epidermin dwurde durch Zugabe von 2 Portionen, bestehend jeweils aus 45 g Amberlite® IRC-50, innerhalb von 1 Stunde zum Eluat auf dieses Harz adsorbiert. Das Harz wurde zum Waschen und Eluieren in eine Säule übertragen.

| Waschen: | 2 l 0,05 N Na-Phosphatpuffer pH 7,0; |
|---|---|
| Elution: | 10 l 0,05 N Na-Phosphat, 1,5 N Kochsalz in Wasser/Methanol 8:2 (v:v), pH 7,0. |

Zur Entsalzung wurde der pH-Wert des Eluats auf 6,0 eingestellt und Epidermin schubweise an Amberlite® XAD-1180 adsorbiert, in 2 Chargen zu jeweils 75 g Trockengewicht innerhalb 1 Stunde. Zum Waschen und Eluieren wurde das Harz in eine Kolonne gepackt.

| Waschen: | 20 l Wasser |
|---|---|
| Elution: | 2,5 l Methanol/0,01 N Salzsäure 9:1 (v:v). |

Nach dem Verdampfen des Lösungsmittels und Gefriertrocknung des Eluats erhielt man 6500 mg einer Substanz, die 5200 mg Epidermin (80 % Reinheit) enthielt. Die Endreinigung erfolgte durch präparative HPLC mit Gradient-Elution:

| Kolonne: | Nucleosil 100 C-18 (10μ) |
|---|---|
| Lösungsmittel: | A: Wasser mit 1 Gew.-% Ameisensäure |
| | B: Methanol/Wasser 8:2 enthaltend 1 % Ameisensäure. |

Nach präparativer HPLC (high performance liquid chromatography) und Gefriertrocknung erhielt man 4,94 g reines Epidermin. Das Antibiotikum erwies sich bei allen damit durchgeführten Tests als einheitlich. Das Flußdiagramm ist in Abb. 10 dargestellt.

Während der Herstellung und Isolierung des Epidermins wurde zur Kontrolle bzw. biologischen Charakterisierung der Plattendiffusionstest angewandt. Näheres hierzu kann aus der EP-A-85 113 908.8 entnommen werden. In dieser Publikation wird der Stamm Staphylococcus epidermidis DSM 3095 nähers definiert.

Die durch den Stamm DSM 3095 erzeugte Kulturbrühe kann auch durch eine solche, die durch den Stamm NCIB 11536 (hinterlegt bei der National Collection of Industrial Bacteria, Aberdeen) erzeugt wurde, ausgetauscht werden, um darin Epidermin zu erzeugen, zu isolieren und es anschließend zu reinigen. Bezüglich der Epiderminproduktion ist aber der Stamm NCIB 11536 dem Stamm DSM 3095 unterlegen.

Epidermin wirkt als Antibiotikum gegen Hautinfektionen wie Ekzeme, Impetigo, Cellulitis und Akne.

**Patentansprüche**

1. Verfahren zur Isolierung von Epidermin aus einer Kulturbrühe oder einem Kulturfiltrat eines Staphylococcus epidermidis-Stammes und zur Reinigung dieses Stoffes, dadurch gekennzeichnet, daß
   a.) das Kulturfiltrat oder die Kulturbrühe in ein Styrol-divinyl-copolymerisat eingetragen,
   b.) die Wirkkomponente vom Harz durch Elution mit Methanol/verdünnter Salzsäure losgelöst,
   c.) das Eluat auf einen pH-Wert von 5,3 bis 5,8 eingestellt,
   d.) das Eluat auf einen schwachen Kationenaustauscher gebracht wird,
   e.) nichtgebundene Substanzen anschließend mit einer Pufferlösung bei pH 7 ausgewaschen werden,
   f.) die Wirkkomponente aus dem Kationenaustauscher mit einer Lösung bestehend aus Puffersubstanz, Natriumchlorid und Methanol bei pH 6,0 bis 8,0 eluiert wird und zur Reinigung
   g.) der Wirkstoff des Eluats auf einem Styrol-divinyl-copolymerisat readsorbiert, das Harz zur Entsalzung mit Wasser gewaschen und das Epidermin mit einer Methanol/Essigsäure-Mischung vom Harz gelöst und die Lösung evaporiert bzw. gefriergetrocknet wird, wobei das dabei gewonnene Epidermin noch anschließend zur Feinreinigung einer Hochleistungs-Flüssig-Chromatographie (HPLC) unterworfen werden kann.

2. Verfahren nach Anspruch 1, dadurch gekennzeichnet, daß Epidermin aus der Kulturbrühe oder dem Kulturfiltrat durch Adsorption an ein Styrol-divinyl-copolymerisat extrahiert, der Wirkstoff mit Methanol/0,01 N Salzsäure (9:1 v:v) aus dem Harz freigesetzt, das Eluat, das auf einem pH-Wert zwischen 5,3 und 5,8 eingestellt wurde, auf ein Methacrylsäure-Divinylbenzol-Copolymerisat, wie Amberlite® IRC-50 oder IRC-84, als Kationenaustauscher gebracht wird, wobei der Ionenaustausch in einer Säule oder auch durch schubweises Eintragen erfolgen kann, nichtgebundene Substanzen aus dem Harz mit einem 0,05 N Natriumphosphatpuffer bei pH 7,0 ausgewaschen werden, der Wirkstoff aus dem Harz mit einer Lösung bestehend aus 80 % Natriumphosphatpuffer, 20 % Methanol, die 1,5 N bezüglich Kochsalz ist, bei pH 7 abgelöst, das so gewonnene Eluat auf einen pH-Wert von 6,0 eingestellt und auf ein Styrol-divinyl-copolymerisat-Harz gegeben wird, welches anschließend durch Auswaschen der Salze gereinigt wird, das Epidermin von diesem Harz mit Methanol/50 %-iger Essigsäure (9:1 v:v) losgelöst und das Eluat evaporiert bzw. gefriergetrocknet wird, wobei das so erhaltene Epidermin gewünschtenfalls mittels präparativer Hochleistungs-Flüssig-Chromatographie (HPLC) noch einer Endreinigung unterzogen werden kann.

3. Verfahren nach Anspruch 1 und 2, dadurch gekennzeichnet, daß die Epidermin-Produktion durch eine schubweise Fermentation in einem Bioreaktor bei einer Temperatur von 35 bis 37°C, einer Belüftungsrate von 0,2 bis 0,6 vvm und Rührerrate von 700 bis 1000 U/min. unter Zugabe eines Chlorids, vorzugsweise von Natriumchlorid, in einer Menge bis zu 70 g pro Liter und/oder eines Eisensalzes in einer Menge bis zu 150 $\mu$M erfolgt.

4. Verfahren nach Anspruch 3, dadurch gekennzeichnet, daß C-Quellen und/oder Phosphate und/oder Ammoniak oder Ammoniumsalze kontinuierlich oder diskontinuierlich der Fermentationsbrühe im Verlauf der Fermentation zugeführt werden.

5. Verfahren nach den Ansprüchen 1 bis 4, dadurch gekennzeichnet, daß während des Fermentationsverlaufs diskontinuierlich der Fermentationsbrühe das gebildete Epidermin durch Adsorption an Styrol-divinyl-copolymerisate entzogen wird, wobei die so behandelte Fermentationsbrühe und Biomasse dem Fermenter wieder zugeführt wird.

6. Verfahren nach den Ansprüchen 1 bis 4, dadurch gekennzeichnet, daß während des Fermentationsverlaufes durch kontinuierliche "on-line"-Adsorption an Styrol-divinyl-copolymerisate das gebildete Epidermin der Fermentationsbrühe entzogen wird, wobei das Retentat laufend dem Reaktor wieder zugeführt wird.

7. Verfahren nach Anspruch 1 bis 3, dadurch gekennzeichnet, daß das Kulturmedium 2 bis 4 Gew.-% Fleischextrakt, 1 bis 3 Gew.-% Malzextrakt oder Maltose, Galaktose, Laktose, Glukose oder Mischungen aus Laktose mit Maltose, Galaktose mit Maltose, und 0,25 bis 1 Gew.-% Calciumcarbonat oder 0,25 bis 0,5 Gew.-% Calciumhydroxid des weiteren fakultativ 1 bis 6 Gew.-% Alkalichloride, 0,001 bis 0,002 Gew.-% Eisenionen und Ammoniumsalze entsprechend einer 57 bis 200 m molaren Lösung bei einem pH-Wert zwischen 6 und 7 enthält.

8. Verfahren nach Anspruch 7, dadurch gekennzeichnet, daß das Kulturmedium 3,3 Gew.-% Fleischextrakt, 3 Gew.-% Malzextrakt oder Maltose, 0,37 Gew.-% Calciumhydroxid und, gegebenenfalls, 1 bis 6 Gew.-% Natriumchlorid und/oder Kaliumchlorid, 0,001 bis 0,002 Gew.-% Eisenionen, vorzugsweise in Form von Ferrichlorid oder Ferrosulfat, sowie Ammoniumsalze, vorzugsweise Ammoniumchlorid oder -sulfat entsprechend einer 57 bis 200 m molaren Lösung, gegebenenfalls auch Kalium- oder Natriumdihydrogenphosphat bei einem pH-Wert von 6 bis 7 enhält.

9. Verfahren nach den Ansprüchen 1 bis 8, dadurch gekennzeichnet, daß als Epidermin erzeugender Stamm Staphylococcus epidermidis DSM 3095 verwendet wird.

10. Verfahren nach den Ansprüchen 1 bis 8, dadurch gekennzeichnet, daß als Epidermin erzeugender Stamm Staphylococcus epidermidis NCIB 11536 verwendet wird.

**Claims**

1. Process for isolating epidermin from a culture broth or a culture filtrate of a strain of Staphylococcus epidermidis and for purifying this substance, characterised in that
   a.) the culture filtrate or culture broth is added to a styrene-divinyl copolymer,
   b.) the active component is released from the resin by elution with methanol/diluted hydrochloric acid,
   c.) the eluate is adjusted to a pH of 5.3 to 5.8,
   d.) the eluate is placed on a weak cation exchanger,
   e.) non-bound substances are subsequently washed out with a buffer solution at pH 7,
   f.) the active component is eluted out of the cation exchanger with a solution consisting of buffer substance, sodium chloride and methanol at pH 6.0 to 8.0 and for purification
   g.) the active substance of the eluate is readsorbed on a styrene-divinyl copolymer, the resin is washed with water in order to remove salts and the epidermin is released from the resin with a methanol/acetic acid mixture and the solution is evaporated or freeze-dried, whilst the epidermin thus obtained may subsequently also be subjected to high performance liquid chromatography for extra purification.

2. Process according to claim 1, characterised in that epidermin is extracted from the culture broth or culture filtrate by adsorption on a styrene-divinyl copolymer, the active substance is liberated from the resin using methanol/0.01 N hydrochloric acid (9:1 v:v), the eluate, adjusted to a pH of between 5.3 and 5.8, is placed on a methacrylic acid-divinylbenzene copolymer such as Amberlite® IRC-50 or IRC-84 as cation exchanger, whilst the ion exchange may take place in a column or by introduction in small amounts, non-bound substances are washed out of the resin with a 0.05 N sodium phosphate buffer at pH 7.0, the active substance is liberated from the resin with a solution consisting of 80% sodium phosphate buffer, 20% methanol, which is 1.5 N with regard to common salt, at pH 7, the eluate thus obtained is adjusted to a pH of 6.0 and added to a styrene-divinyl copolymer resin which is subsequently purified by washing out the salts, the epidermin is released from this resin using methanol/50% acetic acid (9:1 v:v) and the eluate is evaporated or freeze-dried, whilst the epidermin thus obtained may if desired be subjected to final purification by preparative high performance liquid chromatography (HPLC).

3. Process according to claims 1 and 2, characterised in that the epidermin production is carried out by batchwise fermentation in a bioreactor at a temperature of 35 to 37°C, a ventilation rate of 0.2 to 0.6 vvm and a stirrer rate of 700 to 1000 rpm with the addition of a chloride, preferably sodium chloride, in a quantity up to 70 g per litre and/or an iron salt in a quantity of up to 150 $\mu$M.

4. Process according to claim 3, characterised in that C-sources and/or phosphates and/or ammonia or ammonium salts are continuously or discontinuously fed into the fermentation liquor during fermentation.

5. Process according to claims 1 to 4, characterised in that during the course of fermentation the epidermin formed is discontinuously removed from the fermentation liquor by adsorption on styrene-divinyl copolymers, whilst the fermentation liquor and biomass thus treated are recycled into the fermenter.

6. Process according to claims 1 to 4, characterised in that during the course of fermentation the epidermin formed is removed from the fermentation liquor by continuous on-line adsorption on styrene-divinyl copolymers, whilst the retained material is continuously recycled into the reactor.

7. Process according to claims 1 to 3, characterised in that the culture medium contains 2 to 4% by weight of meat extract, 1 to 3% by weight of malt extract or maltose, galactose, lactose, glucose or mixtures of lactose and maltose, galactose and maltose, and 0.25 to 1% by weight of calcium carbonate or 0.25 to 0.5% by weight of calcium hydroxide, and also optionally 1 to 6% by weight of alkali metal chlorides, 0.001 to 0.002% by weight of iron ions and ammonium salts corresponding to a 57 to 200 mmolar solution at a pH of between 6 and 7.

8. Process according to claim 7, characterised in that the culture medium contains 3.3% by weight of meat extract, 3% by weight of malt extract or maltose, 0.37% by weight of calcium hydroxide and, optionally, 1 to 6% by weight of sodium chloride and/or potassium chloride, 0.001 to 0.002% by weight of iron ions, preferably in the form of ferric chloride or ferrous sulphate, and ammonium salts, preferably ammonium chloride or sulphate, corresponding to a 57 to 200 mmolar solution, optionally together with potassium or sodium dihydrogen phosphate at a pH of 6 to 7.

9. Process according to claims 1 to 8, characterised in that Staphylococcus epidermidis DSM-3095 is used as the epidermin-producing strain.

10. Process according to claims 1 to 8, characterised in that Staphylococcus epidermidis NCIB-11536 is used as the epidermin-producing strain.

**Revendications**

1. Procédé pour isoler l'épidermine à partir d'un bouillon de culture ou d'un filtrat de culture d'une souche de Staphylococcus epidermidis et pour purifier cette substance, caractérisé en ce que

a) le filtrat de culture ou le bouillon de culture est introduit dans un copolymère styrène-composé divinylique,
b) le composant actif est séparé de la résine par élution avec du méthanol/acide chlorhydrique dilué,
c) l'éluat est porté à un pH de 5,3 à 5,8,
d) l'éluat est amené sur un échangeur de cations faible,
e) les substances non liées sont ensuite séparées par lavage avec une solution tampon à pH 7,
f) le composant actif est élué de l'échangeur de cations avec une solution consistant en une substance tampon, du chlorure de sodium et du méthanol à un pH de 6,0 à 8,0 et pour la purification
g) le principe actif de l'éluat est réadsorbé sur un copolymère styrène-composé divinylique, la résine est lavée à l'eau pour la débarrasser des sels et l'épidermine est séparée de la résine avec un mélange méthanol/acide acétique et la solution est évaporée ou lyophilisée, l'épidermine ainsi obtenue pouvant encore être soumise à une chromatographie liquide à haute performance (HPLC) en vue d'une purification poussée.

**2.** Procédé selon la revendication 1, caractérisé en ce que l'épidermine est extraite du bouillon de culture ou du filtrat de culture par adsorption sur un copolymère styrène-composé divinylique, le principe actif est libéré de la résine avec du méthanol/acide chlorhydrique 0,01 N (9:1 v:v), l'éluat, qui a été porté à un pH compris entre 5,3 et 5,8, est amené sur un copolymère acide méthacrylique-divinylbenzène, tel que l'Amberlite® IRC-50 ou IRC-84, en tant qu'échangeur de cations, l'échange d'ions pouvant se produire dans une colonne ou encore par introduction par lots, les substances non liées étant séparées de la résine par lavage avec un tampon phosphate de sodium 0,05 N à pH 7,0, le principe actif étant séparé de la résine avec une solution consistant en 80% de tampon phosphate de sodium, 20% de méthanol, qui est 1,5 N par rapport au chlorure de sodium, à pH 7, l'éluat ainsi obtenu étant porté à un pH de 6,0 et amené sur une résine de copolymère styrène-composé divinylique qui est nettoyée ensuite par élimination des sels par lavage, l'épidermine étant séparée de cette résine avec du méthanol/acide acétique à 50% (9:1 v:v) et l'éluat étant évaporé ou lyophilisé, l'épidermine ainsi obtenue pouvant, si on le souhaite, être soumise encore à une purification finale par chromatographie liquide à haute performance (HPLC) préparative.

**3.** Procédé selon les revendications 1 et 2, caractérisé en ce que la production d'épidermine est accomplie par une fermentation par lots dans un bioréacteur à une température de 35 à 37 °C, un débit d'aération de 0,2 à 0,6 vvm et une vitesse d'agitation de 700 à 1000 tr/min avec addition d'un chlorure, de préférence de chlorure de sodium, en une quantité pouvant atteindre 70 g par litre et/ou d'un sel de fer en une quantité pouvant atteindre 150 $\mu$M.

**4.** Procédé selon la revendication 3, caractérisé en ce que des sources de C et/ou des phosphates et/ou de l'ammoniaque ou des sels d'ammonium sont introduits de manière continue ou discontinue dans le bouillon de fermentation au cours de la fermentation.

**5.** Procédé selon les revendications 1 à 4, caractérisé en ce que, pendant le déroulement de la fermentation, l'épidermine formée est retirée de manière discontinue du bouillon de fermentation par adsorption sur des copolymères styrène-composé divinylique, le bouillon de fermentation et la biomasse ainsi traités étant renvoyés dans le fermenteur.

**6.** Procédé selon les revendications 1 à 4, caractérisé en ce que, pendant le déroulement de la fermentation, l'épidermine formée est retirée du bouillon de fermentation par adsorption "en ligne" continue sur des copolymères styrène-composé divinylique, le rétentat étant renvoyé en continu dans le réacteur.

**7.** Procédé selon les revendications 1 à 3, caractérisé en ce que le milieu de culture contient 2 à 4% en poids d'extrait de viande, 1 à 3% en poids d'extrait de malt ou de maltose, de galactose, de lactose, de glucose ou de mélanges de lactose et de maltose, de galactose et de maltose, et 0,25 à 1% en poids de carbonate de calcium ou 0,25 à 0,5% en poids d'hydroxyde de calcium, et en outre de manière facultative 1 à 6% en poids de chlorures alcalins, 0,001 à 0,002% en poids d'ions fer et des sels d'ammonium en une quantité correspondant à une solution 57 à 200 fois mmolaire à un pH situé entre 6 et 7.

**8.** Procédé selon la revendication 7, caractérisé en ce que le milieu de culture contient 3,3% en poids d'extrait de viande, 3% en poids d'extrait de malt ou de maltose, 0,37% en poids d'hydroxyde de calcium et, éventuellement, 1 à 6% en poids de chlorure de sodium et/ou de chlorure de potassium, 0,001 à 0,002% en poids d'ions fer, de préférence sous forme de chlorure ferrique ou de sulfate ferreux, ainsi que des sels d'ammonium, de préférence du chlorure d'ammonium ou du sulfate d'ammonium, en une quantité correspondant à une solution 57 à 200 fois mmolaire, éventuellement aussi du dihydrogénophosphate de potassium ou de sodium à un pH de 6 à 7.

**9.** Procédé selon les revendications 1 à 8, caractérisé en ce que l'on utilise comme souche productrice d'épidermine la souche de Staphylococcus epidermidis DSM 3095.

**10.** Procédé selon les revendications 1 à 8, caractérisé en ce que l'on utilise comme souche productrice d'épidermine la souche de Staphylococcus epidermidis NCIB 11536.

ABB.1.

ABB.5.

ABB. 2.

EP 0 350 810 B1

Verbrauch $N_2$ [g/l]

Verbrauch Phosphate [mM]

Fermentierter Zucker total

log Anzahl lebender Zellen

Epidermin Konz. [mg/l]

$10^{11}$

300

$10^{10}$

200

$10^9$

100

$10^8$

60

60

50

50

40

40

30

30

20

20

10

10

4

3

2

1

ABB.3.

0   25   50   70   100   125   150

Zeit [h]

ABB. 4.

ABB.6.

ABB.7.

ABB.8.

ABB.9.

Washen und Elution

Ammoniak + Phosphate

Glu-cose

Zufuhr

XAD-1180

Sieb

Eluat

Filtrat

Retentat

Cross-Flow-Filtrationseinheit

"Batch" IRC Adsorption

IRC-50

Eluat

"Batch" XAD 1180 Adsorption

XAD-1180

Eluat

Evaporation und Lyophilisation

Präparative HPLC

ABB.10.

EP 0 350 810 B1